# EUROPEAN PATENT SPECIFICATION

(11) **EP 1 894 909 B1**
(45) Date of publication and mention of the grant of the patent: **05.04.2017**
(21) Application number: 07023387.9
(22) Date of filing: 06.02.2002
(51) Int. Cl.: C07C 15/02, C07C 2/66, C07C 6/12, C07C 15/073, C07C 15/085

(54) **Production of alkylaromatic compounds**
Herstellung von alkylaromatischen Verbindungen
Production de composés alkyl-aromatiques

(30) Priority: 07.02.2001 US 267035 P
(43) Date of publication of application: 05.03.2008
(62) Divisional of application: 02707745.2
(73) Proprietor: Badger Licensing LLC, Boston, MA 02111 (US)
(72) Inventor: Chen, Shiou-Shan, Winchester MA 01890 (US); Hwang, Shyh-Yuan H., Needham MA 02494 (US)
(74) Representative: Denness, James Edward

(56) References cited:
- US-A- 5 003 119
- US-A- 6 043 402

## Description

The present invention relates generally to improvements in the production of alkyl aromatic compounds, particularly cumene and ethylbenzene, utilizing alkylation and transalkylation in novel series or combination configurations to achieve significant improvements in process efficiencies.

### BACKGROUND OF THE INVENTION

### Cumene and Cumene Production

Cumene is an aromatic compound. It is a clear liquid at ambient conditions. High purity cumene is conventionally manufactured from propylene and benzene. Cumene is used today primarily as a feed in manufacturing the products phenol and acetone, which are two important petrochemicals with many uses in the chemical and polymer industries. Global cumene production in 1998 was about 7 million metric tons.

Cumene was first synthesized in large quantities during World War II as an aviation gasoline. It has a high heating value and a high octane number, but it is not economically competitive today as a fuel. Its presence in gasoline is now incidental, being an inevitable minor reaction product of refinery processes such as catalytic reforming and steam cracking.

Production of cumene was considered a rather conventional and routine business for many years, but recently has generated considerable excitement for two reasons. First, the demand for phenol for manufacturing polycarbonates is accelerating rapidly owing to the broadening applications of polycarbonates in the electronic, healthcare, and automobile industries. Second, successful development and commercialization of the zeolite-based alkylation technology for the isopropylation of benzene to cumene has rendered obsolete the older processes which were based on solid phosphoric acid and aluminum chloride. Within a period of just over two years during 1996-98, over one half of the cumene capacity in the world was converted to the new zeolite technologies.

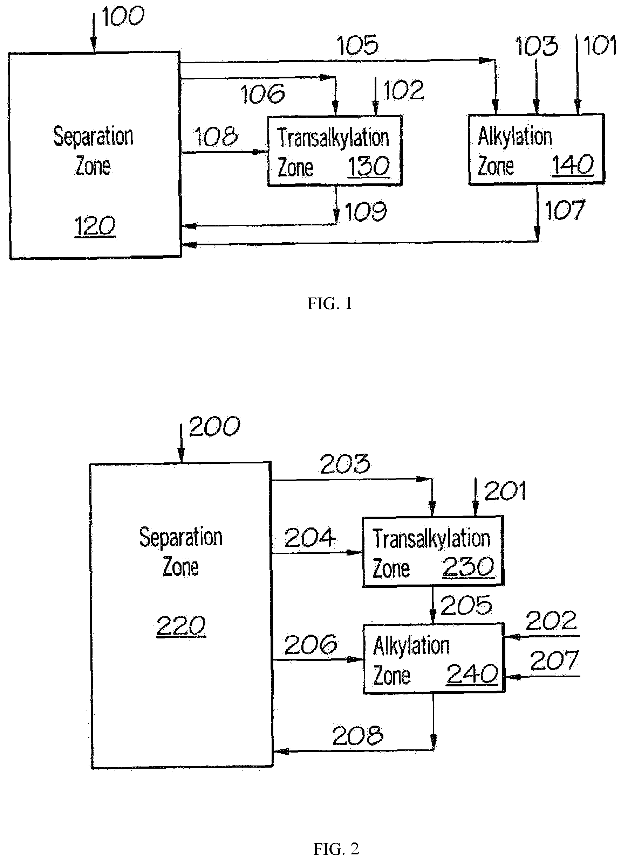

New zeolite-based cumene technologies developed by Mobil/Badger, Dow/Kellogg, and UOP carry out the alkylation of benzene and propylene in liquid phase in the presence of a solid acidic zeolite catalyst. A process developed by CDTech achieves the alkylation of benzene and propylene in mixed phases in a catalytic distillation column packed with both distillation devices and bales of zeolite catalysts. Figure 1 is a simplified representation of the zeolite-based cumene technologies. All of these zeolite-based cumene technologies utilize a separate transalkylation zone which is operated in parallel with the alkylation zone, to react a mixture of benzene and the polyisopropylbenzene alkylation byproducts to form additional cumene in liquid phase in the presence of a solid acidic catalyst. A separation zone is utilized to recover the unreacted benzene and polyisopropylbenzenes for recycle, and to isolate the desired cumene product.

### Ethylbenzene and Ethylbenzene Production

Ethylbenzene is a commodity chemical currently used mostly for the production of styrene. Global ethylbenzene production in 1998 was about 19 million metric tons. Ethylbenzene may be prepared by a number of different chemical processes, but present commercial ethylbenzene production is dominated by zeolite-based technologies. The first zeolite-based ethylbenzene process, developed jointly by Mobil and Badger in the early 1980s, utilizes a combination of vapor phase alkylation of benzene with ethylene and vapor phase trans-alkylation of a benzene and polyethylbenzene mixture. Both the alkylation and transalkylation steps are carried out in the presence of solid acidic ZSM-5 catalysts.

Several liquid-phase zeolite-based ethylbenzene technologies were developed in the late 1980s and in the 1990s by UOP/Lummus and Mobil/Badger. Alkylation of benzene with ethylene and transalkylation of mixtures of benzene and polyethylbenzenes are carried out in liquid phase in the presence of solid acidic zeolite catalysts. Catalysts that can be used for alkylation of benzene with ethylene and for transalkylation of benzene and polyethylbenzenes in at least partial liquid phase include zeolite beta, zeolite Y, ZSM-5, PSH-3, ITQ-2, ZSM-12, MCM-22, MCM-36, MCM-49, MCM-56, MCM-58, MCM-68, faujasite, mordenite, porous crystalline magnesium silicates, and tungstate modified zirconia.

Processes for the production of ethylbenzene over intermediate-pore size zeolites are described in U.S. Patents 3,751,504 (Keown); 4,547,605 (Kresge); and 4,016,218 (Haag). U.S. Patents 4,169,111 (Wight) and 4,459,426 (Inwood) disclose production of ethylbenzene over large-pore size zeolites such as zeolite Y. A process for ethylbenzene production over zeolite ZSM-12 is described in U.S. Patent 3,755,483 (Burress). Liquid phase synthesis of ethylbenzene with zeolite beta is described in U.S. Patent 4,891,458.

To minimize the formation of polyalkylaromatics and other undesired impurities (e.g., oligomers of the olefin), production of alkylaromatics such as ethylbenzene and cumene typically operates with relatively high (excess) mole ratios of aromatic (e.g., benzene) to olefin (e.g., ethylene or propylene) in the alkylation reactor feed. Zeolite-based alkylaromatic processes generally operate at aromatic to olefin feed molar ratios of three or above, while aluminum chloride-based processes often operate at aromatic to olefin molar ratios of three and below. In both cases, however, the polyalkylaromatics are produced at sufficiently high levels that it would be prohibitively expensive to simply dispose of them as low value byproducts. Instead, these polyalkylated aromatics are typically reacted further with feed aromatic to form additional monoalkylate via transalkylation reactions.

In the case of the Mobil/Badger vapor phase ethylbenzene process mentioned above, the transalkylation reaction may take place in the alkylation reactor or in a separate transalkylation reactor. U.S. Patents 5,902,917 (Collins) and 6,096,935 (Schulz) describe processes for the production of alkylaromatics wherein a feedstock is first fed to a transalkylation zone and the entire effluent from the transalkylation zone is then cascaded directly into an alkylation zone along with an olefin alkylating agent.

US 5,003,119 discloses a process for the manufacture of alkylbenzenes wherein a feed of fresh and recycle benzene and fresh olefin are reacted in the presence of an alkylation catalyst having at least two stages.

Conventionally, relatively high molar ratios of aromatic (e.g., benzene) to olefin (e.g., ethylene or propylene) have been used successfully commercially in the production of alkylaromatics (e.g., ethylbenzene or cumene) to minimize the formation of polyalkylaromatics and other undesired impurities (e.g., oligomers of the olefins). The disadvantage of using high molar ratios of aromatic to olefin, however, is that the recovery and the subsequent circulation (re-use) of the unreacted aromatics consumes very substantial amounts of energy which increases the production cost of the desired alkylaromatics.

The recovery and circulation of large amounts of unreacted aromatics also requires larger capacity separation equipment (usually distillation columns) and larger pumps, both of which increase capital cost of the plant, and thus also increase the cost of production.

It is therefore of crucial interest to minimize the amount of excess aromatics that is used and needs to be recovered and subsequently circulated in order to minimize the production cost. It is of even more importance today in the production of highly competitive commodity chemicals (e.g., ethylbenzene and cumene) which are produced and traded globally, and at a time when the energy costs are high. Low aromatics circulation results in lower energy consumption, lower capital investment and thus a more efficient plant. This in turn enables a producer to establish itself as a low cost producer in a favorable (competitive) marketing position.

Because of these disadvantages and limitations of the prior art processes, it is desired to provide improved processes and apparatus for the production of alkylaromatic compounds. In this invention, two reaction configurations are provided which have been found to significantly reduce the total aromatic circulation, compared with prior art processes, at all aromatic to olefin ratios.

### OBJECTS OF THE INVENTION

Accordingly, a general object of this invention is to provide an improved process for the production of alkylaromatic compounds, particularly cumene and ethylbenzene.

More specifically, it is a principal object of this invention to provide a process for the production of alkylaromatic compounds using less circulation of aromatics than in conventional processes.

It is a further principal object of this invention to provide a process for the production of alkylaromatic compounds which uses relatively low circulation of aromatics without increasing the production of unwanted byproducts, specifically polyalkylaromatics.

Another specific object of this invention is to provide a process using a reaction zone for the production of alkylaromatic compounds comprising an alkylation unit and a transalkylation unit in a particular series configuration.

Yet a further specific object of this invention is to prepare alkylaromatic compounds, such as cumene and ethylbenzene, utilizing the processes and apparatus described herein.

Other objects and advantages of the present invention will in part be obvious and will in part appear hereinafter. The invention accordingly comprises, but is not limited to, the processes and related apparatus, involving the several steps and the various components, and the relation and order of one or more such steps and components with respect to each of the others, as exemplified by the following description and the accompanying drawings. Various modifications of and variations on the processes and apparatus as herein described will be apparent to those skilled in the art, and all such modifications and variations are considered within the scope of the invention.

### SUMMARY OF THE INVENTION

The present invention relates generally to improvements in alkylation and transalkylation processes in the production of alkylaromatic compounds, for example cumene and ethylbenzene, utilizing a reaction section and a separation section. The reaction section configuration provided in this invention requires significantly less total aromatics distillation and circulation (recycle) than do the conventional configurations of both parallel alkylation/transalkylation reactors and the transalkylator-alkylator cascaded configuration described in U.S. Patents 5,902,917 (Collins) and 6,096,935 (Schulz), even when operated at the same alkylator feed aromatics to olefin molar ratio, thereby reducing the capital and utility costs of producing the desired alkylaromatics.

In a first embodiment of the improved alkylaromatic production processes and apparatus according to this invention, the reaction section comprises an alkylation zone and a transalkylation zone configured to operate in a novel series arrangement. In this first embodiment, two or more separate feeds respectively consisting essentially of fresh and recycle aromatics and fresh olefin, or one or more at least partially combined feeds of aromatics and/or olefin, are sent to an alkylation zone where the aromatics and olefin are reacted in the presence of an alkylation catalyst in the alkylation zone. The effluent from the alkylation zone is sent to a transalkylation zone together with recycled polyalkylaromatics for the production of additional alkylaromatics. The alkylation and the transalkylation zones can be housed in the same vessel or in different vessels.

The alkylation is carried out substantially adiabatically in at least partial liquid phase, at temperatures between about 150°F (66°C) and 900°F (482°C) and at pressures between about 150 psig (1034 kPAg) and 2000 psig (13788 kPAg), over one or more beds of suitable alkylation catalyst(s) consisting essentially of at least one solid acid oxide selected from the group consisting of: MCM-22, MCM-36, MCM-49, MCM-56, MCM-58, MCM-68, and tungstate modified zirconia. The overall molar ratio of aromatics to olefin fed to the alkylation zone is between about 1:1 and 20:1. The olefin fed to the alkylation zone is essentially completely reacted with the aromatics feed. The alkylation zone can be housed in one or more vessels.

The transalkylation is carried out substantially adiabatically in at least partial liquid phase, at temperatures between about 150°F (66°C) and 900°F (482°C) and at pressures between about 150 psig (1034 kPAg) and 2000 psig (13788 kPAg), over one or more beds of suitable transalkylation catalyst(s) consisting essentially of at least one solid acid oxide selected from the group consisting of: zeolite beta, zeolite Y, ZSM-5, PSH-3, ITQ-2, ZSM-12, MCM-22, MCM-36, MCM-49, MCM-56, MCM-58, MCM-68, faujasite, mordenite, porous crystalline magnesium silicates, and tungstate modified zirconia. The overall weight ratio of aromatics to polyaromatics fed to the transalkylation zone is between about 0.2:1 and 20:1. The transalkylation zone can be housed either in one or more separate vessels or, alternatively, in the same vessel or vessels where the alkylation zone is housed.

This process and apparatus may further comprise a separation zone wherein unreacted aromatic and polyalkylaromatic compounds are recovered and recycled, and the desired alkylaromatic product, for example cumene or ethylbenzene, is isolated.

### BRIEF DESCRIPTION OF THE DRAWINGS

Fig. 1 is a schematic representation of a first prior art process, as discussed above, for the production of alkylaromatic compounds in which separate alkylation and transalkylation reactors are configured in a conventional parallel mode.
Fig. 2 is a schematic representation of a second prior art process, as discussed above, for the production of alkylaromatic compounds, a cascaded configuration in which the entire effluent from the transalkylation zone is cascaded directly into an alkylation zone along with an olefin.
Fig. 3 is a schematic representation of a first embodiment of the present invention wherein the reaction section comprises an alkylation zone and a transalkylation zone configured to operate in series.
Fig. 4 is a graph comparing total benzene circulation for production of cumene at varying alkylator benzene/propylene feed ratios for the prior art reactor configurations of Figs. 1 and 2 with those for the reactor configuration of Fig. 3.

### DETAILED DESCRIPTION OF PREFERRED EMBODIMENTS

The novelty of the present invention can best be understood by comparison with and contrast to two important prior art approaches to producing alkylaromatic compounds.

A first familiar conventional approach to the production of alkylaromatic compounds, wherein separate alkylation and transalkylation reactors are used and operated in parallel mode, is illustrated schematically in Fig. 1. As shown in Fig. 1, the fresh aromatics feed can enter the process via line 100 to the separation zone 120, via line 101 into the alkylation zone 140, or via line 102 into the transalkylation zone 130. Part of the aromatic feed recovered from the separation zone 120 is sent to the alkylation zone 140 via line 105. A second part of the aromatic feed recovered in the separation zone 120 is sent to the transalkylation zone 130 via line 106. Fresh olefin feed is introduced into the alkylation zone 140 via line 103, and the effluent from the alkylation zone 140 is sent to the separation zone 120 via line 107. The polyalkylaromatics product recovered in the separation zone 120 is sent to the transalkylator 130 via line 108. The effluent from the transalkylation zone 130 is sent to the separation zone 120 via line 109.

A second familiar conventional approach to the production of alkylaromatic compounds, utilizing a cascaded process as described in U.S. Patents 5,902,917 (Collins) and 6,096,935 (Schulz), is illustrated schematically in Fig. 2. As shown in Fig. 2, the fresh aromatics feed can enter the process either via line 200 to the separation zone 220, via line 201 to the transalkylation zone 230, or via line 202 into the alkylation zone 240. A first part of the aromatic feed recovered from the separation zone 220 is sent to the transalkylation zone 230 via line 203. A second part of the aromatic feed recovered from the separation section 220 is introduced into the alkylation zone 240 via line 206. The polyalkylaromatic product recovered from the separation zone 220 is sent to the transalkylation zone 230 via line 204. The effluent from the transalkylation zone 230 is sent to the alkylation zone 240 via line 205. The fresh olefin feed to the process is introduced into the alkylation zone 240 via line 207 whereas the effluent from the alkylation zone 240 is sent to the separation zone 220 via line 208.

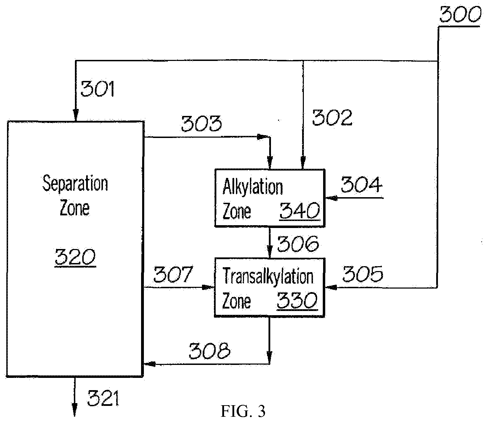

In contrast to the prior art configurations, a first embodiment of the present invention wherein the reaction section comprises an alkylation zone and a transalkylation zone configured to operate in series is illustrated schematically in Fig. 3. As shown in Fig. 3, the fresh aromatics feed 300 enters via line 302 into the alkylation zone 340. Additionally, fresh aromatics feed can also enter the process either via line 301 into separation zone 320, or, alternatively, via line 305 into the transalkylation zone 330. The aromatic feed recovered from separation zone 320 is introduced into the alkylation zone 340 via line 303, and the fresh olefin feed enters the process via line 304 into the alkylation zone 340. The effluent from the alkylation zone 340 is sent to the transalkylation zone 330 via line 306. The polyalkylaromatic product recovered in separation zone 320 is introduced into the transalkylation zone 330 via line 307. The effluent from the transalkylation zone 330 is sent to separation zone 320 via line 308. The monoalkylated product is removed from the separation zone 320 via line 321.

As seen in Fig. 3, the present invention comprises the following process steps:
(a) introducing into an alkylation zone by two or more individual component feeds or one or more at least partly combined feeds, a reaction mixture comprising fresh and recycle aromatics and fresh olefin, wherein the molar ratio of aromatics to olefin in the mixture is in excess of 1:1, and also wherein the alkylation zone includes a suitable alkylation catalyst(s) comprising at least one solid acid oxide selected from the group consisting of MCM-22, MCM-36, MCM-49, MCM-56, MCM-58, MCM-68 and tungstate modified zirconia;
(b) contacting the aromatic/olefin mixture with the alkylation catalyst(s) under sufficient alkylation conditions to react essentially all the olefins in the mixture to monoalkylated aromatics and polyalkylated aromatics, to produce an effluent from the alkylation zone comprising monoalkylated and polyalkylated aromatics and the unreacted aromatics;
(c) introducing a feed into a transalkylation zone, the feed comprising the effluent from the alkylation zone, recycled polyalkylaromatics, and possibly additional aromatics, wherein the transalkylation zone includes a suitable transalkylation catalyst;
(d) contacting the feed to the transalkylation zone with the transalkylation catalyst under sufficient transalkylation conditions to react at least a part of the aromatics and the polyalkylated aromatics in the feed to additional monoalkylated aromatics to produce an effluent from the transalkylation zone comprising the desired mono-alkylated aromatics and the unreacted aromatics and polyalkylated aromatics; and,
(e) introducing the transalkylation zone effluent into a separation zone wherein the desired monoalkylated aromatics product is isolated and recovered and the unreacted aromatics and polyalkylated aromatics are recovered and recycled.

The alkylation step of the present invention may be carried out in at least partial liquid phase at temperatures between about 150°F (66°C) and 900°F (482°C) and at pressures between about 150 psig (1034 kPAg) and 2000 psig (13788 kPAg), over one or more beds of suitable alkylation catalyst(s) comprising at least one solid acid oxide selected from the group consisting of: MCM-22, MCM-36, MCM-49, MCM-56, MCM-58, MCM-68, and tungstate modified zirconia. The overall molar ratio of aromatics to olefin fed to the alkylation zone may be between about 1:1 to 20:1. The olefin fed to the alkylation zone is essentially completely reacted with the aromatics feed. The alkylation zone can be housed in one or more vessels. Each alkylation vessel can have one or more catalyst beds containing the same or different alkylation catalysts or catalyst mixtures. Part of the total effluent from the alkylation zone may be recycled back to some or all of the alkylation catalyst beds, with or without cooling, for temperature control purposes.

MCM-22 catalyst and its use to catalyze the synthesis of alkylaromatics are described in U.S. Patents 4,954,325; 4,992,606; 5,077,445; and 5,334,795. MCM-36 and its use in the synthesis of alkylaromatics are described in U.S. Patents 5,250,277; 5,292,698; and 5,258,565. MCM-49 and its use in the synthesis of alkylaromatics are described in U.S. Patents 5,236,575; 5,493,065; and 5,371,310. MCM-56 and its use to catalyze the synthesis of alkylaromatics are described in U.S. Patents 5,362,697; 5,453,554; 5,557,024; and 6,051,521. MCM-58 and its use for the production of alkylaromatics are described in U.S. Patents 5,437,855 and 5,569,805. MCM-68 and its use for the production of alkylaromatics are described in U.S. Patent 6,049,018. The use of tungstate modified zirconia to catalyze the synthesis of alkylaromatics is described in U.S. Patent 5,563,311.

The transalkylation step of this first embodiment of the present invention is carried out in at least partial liquid phase at temperatures between about 150°F and 900°F and at pressures between about 150 and 2000 psig, over one or more beds of transalkylation catalyst(s) comprising at least one solid acid oxide selected from the group consisting of: zeolite beta, zeolite Y, ZSM-5, PSH-3, ITQ-2, ZSM-12, MCM-22, MCM-36, MCM-49, MCM-56, MCM-58, MCM-68, faujasite, mordenite, porous crystalline magnesium silicates, and tungstate modified zirconia. The overall weight ratio of aromatics to polyalkylaromatics fed to the transalkylation zone may be between about 0.2:1 and 20:1. The transalkylation zone can be housed either in one or more separate vessels or, alternatively, in the same vessel or vessels where the alkylation zone is housed. The transalkylation zone can have one or more catalyst beds containing the same or different transalkylation catalysts or catalyst mixtures.

Zeolite beta catalyst is described in U.S. Patent 3,308,069 and Re. 28,341. Different versions of zeolite Y are described in U.S. Patents 3,130,007; 3,293,192; 3,449,070; and 3, 442,795. ZSM-5 is described in detail in U.S. Patents 3,702,886 and Re. 29,948. ZSM-12 is described in U.S. Patent 3,832,449. PSH-3 is disclosed in U.S. Patent 4,439,409. ITQ-2 is disclosed in U.S. Patent No. 6,231,751.

The following examples will further illustrate the practice and advantages of the present invention.

### Example 1

For production of cumene, computer simulations were carried out to determine the required total benzene circulation at different alkylator feed benzene to propylene molar ratios. The following reactor configurations were considered: (1) the conventional parallel alkylator and transalkylator reactor configuration (Fig. 1); (2) the transalkylator-alkylator cascaded configuration provided in U.S. Patents 5,902,917 (Collins) and 6,096,935 (Schulz) (Fig. 2); (3) the configuration of this inventionalkylator and transalkylator in series configuration (Fig. 3).

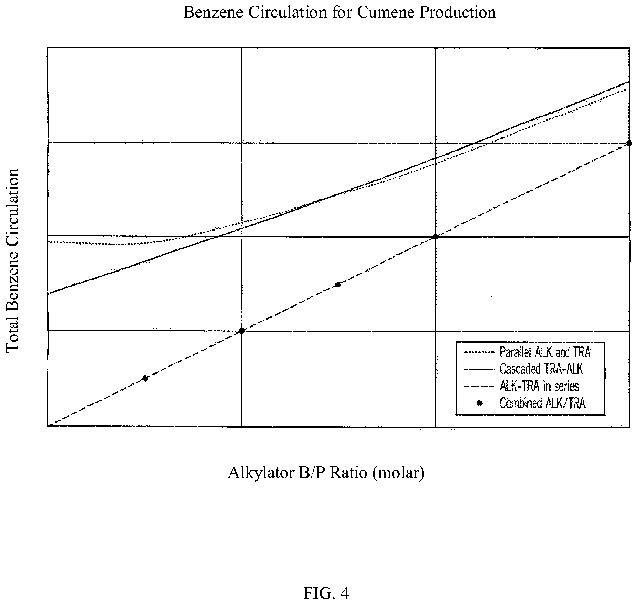

The weight ratio of benzene to polyisopropylbenzenes in the transalkylator feed in the first two reactor configurations considered above was 2. As shown in Figure 4, the reactor configuration provided in the present invention requires significantly less benzene circulation than either the conventional parallel alkylatortransalkylator configuration (parallel ALK and TRA -- dotted line) or the cascaded transalkylator-alkylator configuration (cascaded TRA-ALK -- solid line), thereby confirming the superiority of the reactor embodiment of this invention (ALK-TRA in series configuration -- dashed line).

### Example 2

An alkylation reactor and transalkylation reactor were configured in a series configuration in accordance with Fig. 3 of the present invention. An MCM-22 type catalyst provided by ExxonMobil Chemical Company (identified as MC-1124) was loaded into the alkylation reactor. The amount of the catalyst loaded in the rector was 60 grams. A feed comprising 30 grams per hour (0.71 moles/hr) of propylene and 110 grams per hour (1.41 moles/hr) of benzene was introduced into the alkylation reactor. The alkylation reactor inlet temperature was maintained at 262°F (128°C). The alkylation reactor outlet temperature was maintained at about 284°F (140°C) by recycling part of the alkylation reactor effluent back to the inlet of the alkylation reactor. The alkylation reactor pressure was maintained at about 370 psig (2551 kPAg).

Another MCM-22 type catalyst provided by ExxonMobil Chemical Company (identified as MC-1122) was loaded into the transalkylation reactor. The amount of the catalyst loaded in the reactor was 15 grams. A feed comprising 17 grams per hour of alkylator effluent from the above alkylation reactor and 4 grams per hour of diisopropylbenzenes recovered from alkylator effluent was introduced into the transalkylation reactor. No fresh benzene was fed to the transalkylator zone.) The transalkylation reactor inlet temperature was maintained at 320°F (160°C), and the transalkylation reactor pressure was maintained at about 370 psig (2551 kPAg). A comparison of diisopropylbenzenes in the alkylator effluent, the recovered diisopropylbenzenes, and the transalkylator effluent, as listed in TABLE 1 below, shows that substantially all of the diisopropylbenzene byproduct produced in the alkylator were reacted to monoalkylaromatic compound in the transalkylator.

**TABLE 1**

| Compound | In alkylator effluent, g/hr | In recovered DIPB, g/hr | In transalkylator effluent g/hr |
|---|---|---|---|
| Benzene | 8.4 | 0.0 | 7.4 |
| Cumene | 6.9 | 0.0 | 9.3 |
| Diisopropylbenzenes | 1.3 | 4.0 | 4.0 |

## Claims

1. A process for producing a monalkylated aromatic product in a reactor having an alkylation zone in series with a transalkylation zone, the process comprising the following process steps:
(a) introducing into the alkylation zone by two or more individual component feeds or one or more at least partly combined feeds, a reaction mixture comprising fresh and recycle aromatics and fresh olefin, wherein the molar ratio of aromatics to olefin in the mixture is in excess of 1:1, and also wherein the alkylation zone includes a suitable alkylation catalyst(s) comprising at least one solid acid oxide selected from the group consisting of MCM-22, MCM-36, MCM-49, MCM-56, MCM-58, MCM-68 and tungstate modified zirconia;
(b) contacting the aromatic/olefin mixture with the alkylation catalyst(s) under sufficient alkylation conditions to react essentially all the olefins in the mixture to monoalkylated aromatics and polyalkylated aromatics, to produce an effluent from the alkylation zone comprising monoalkylated and polyalkylated aromatics and the unreacted aromatics;
(c) introducing a feed into a transalkylation zone, the feed comprising the effluent from the alkylation zone, recycled polyalkylaromatics, and optionally additional aromatics, wherein the transalkylation zone includes a suitable transalkylation catalyst;
(d) contacting the feed to the transalkylation zone with the transalkylation catalyst under sufficient transalkylation conditions to react at least a part of the aromatics and the polyalkylated aromatics in the feed to additional monoalkylated aromatics to produce an effluent from the transalkylation zone comprising the desired mono-alkylated aromatics and the unreacted aromatics and polyalkylated aromatics; and,
(e) introducing the transalkylation zone effluent into a separation zone wherein the desired monoalkylated aromatics product is isolated and recovered and the unreacted aromatics and polyalkylated aromatics are recovered and recycled.

2. The process according to claim 1 wherein the alkylation reaction of step (b) is carried out in at least partial liquid phase at temperatures between 150°F (66°C) and 900°F (482°C) and at pressures between 150 psig (1034 kPag) and 2000 psig (13788 kPag).

3. The process of claim 1 wherein overall molar ratio of aromatics to olefin fed to the alkylation zone is between 1:1 to 20:1 such that the olefin fed to the alkylation zone is essentially completely reacted with the aromatics feed.

4. The process according to claim 1 wherein the transalkylation reaction of step (d) is carried out in at least partial liquid phase at temperatures between 150°F (66°C) and 900°F (482°C) and at pressures between 150 psig (1034 kPag) and 2000 psig (13788 KPag).

5. The process according to claim 1 wherein the transalkylation catalyst(s) comprises at least one solid acid oxide selected from the group consisting of: zeolite beta, zeolite Y, ZSM-5, PSH-3, ITQ-2, ZSM-12, MCM-22, MCM-36, MCM-49, MCM-56, MCM-58, MCM-68, faujasite, mordenite, porous crystalline magnesium silicates, and tungstate modified zirconia.

6. The process according to claim 1 wherein the overall weight ratio of aromatics to polyalkylaromatics fed to the transalkylation zone is between 0.2:1 and 20:1.

7. The process of claims 1-6 wherein the aromatics are benzene.

8. The process of claims 1-6 wherein the olefin are selected from the group consisting of ethylene and propylene.

9. The process of claims 1-6 wherein the monoalkylaromatics are selected from the group of ethylbenzene and cumene.

10. The process of claims 1-6 wherein the polyalkylaromatics are selected from polyethylbenzene and polyisopropylbenzene.

## Patentansprüche

1. Verfahren zur Herstellung von monoalkyliertem aromatischen Produkt in einem Reaktor mit einer Alkylierungszone in Reihe mit einer Transalkylierungszone, wobei das Verfahren die folgenden Verfahrensschritte umfasst:
(a) Einbringen einer Reaktionsmischung, die frische und rückgeführte Aromaten und frisches Olefin umfasst, wobei das molare Verhältnis von Aromaten zu Olefin in der Mischung mehr als 1:1 beträgt, durch zwei oder mehr Einzelkomponenten-Einsatzmaterialströme oder ein oder mehrere mindestens teilweise kombinierte Einsatzmaterialströme, in die Alkylierungszone, und wobei die Alkylierungszone ferner einen geeigneten Katalysator (geeignete Katalysatoren) umfassend mindestens ein saures Feststoff-Oxid ausgewählt aus der Gruppe bestehend aus MCM-22, MCM-36, MCM-49, MCM-56, MCM-58, MCM-68 und Wolframat-modifiziertes Zirkonoxid einschließt;
(b) Inkontaktbringen der Aromaten/Olefin-Mischung mit dem Alkylierungskatalysator (den Alkylierungskatalysatoren) unter für die Alkylierung ausreichenden Bedingungen, um im Wesentlichen die gesamten Olefine in der Mischung zu monoalkylierten Aromaten und polyalkylierten Aromaten umzusetzen, um einen Abstrom aus der Alkylierungszone herzustellen, der monoalkylierte und polyalkylierte Aromaten und die unumgesetzten Aromaten umfasst;
(c) Einbringen eines Einsatzmaterials in eine Transalkylierungszone, wobei das Einsatzmaterial den Abstrom aus der Alkylierungszone, rückgeführte Polyalkylaromaten und gegebenenfalls weitere Aromaten umfasst, wobei die Transalkylierungszone einen geeigneten Transalkylierungskatalysator einschließt;
(d) Inkontaktbringen des Einsatzmaterials in der Transalkylierungszone mit dem Transalkylierungskatalysator unter für die Transalkylierung ausreichenden Bedingungen, um mindestens einen Teil der Aromaten und die polyalkylierten Aromaten in dem Einsatzmaterial zu weiteren monoalkylierten Aromaten umzusetzen und einen Abstrom aus der Transalkylierungszone herzustellen, der die gewünschten monoalkylierten Aromaten und die unumgesetzten Aromaten und polyalkylierte Aromaten umfasst; und
(e) Einbringen des Abstroms aus der Transalkylierungszone in eine Abtrennzone, in der das gewünschte Produkt von monoalkylierten Aromaten abgetrennt und gewonnen und unumgesetzte Aromaten und polyalkylierte Aromaten gewonnen und rückgeführt werden.

2. Verfahren nach Anspruch 1, bei dem die Alkylierungsreaktion von Schritt (b) in zumindest teilweise flüssiger Phase bei Temperaturen zwischen 150°F (66°C) und 900°F (482°C) und Drücken zwischen 150 psig (1034 kPaÜ) und 2000 psig (13788 kPaÜ) durchgeführt wird.

3. Verfahren nach Anspruch 1, bei dem insgesamt das molare Verhältnis von Aromaten zu Olefin, das in die Alkylierungszone eingespeist wird, zwischen 1:1 und 20:1 liegt, sodass das in die Alkylierungszone eingespeiste Olefin im Wesentlichen vollständig mit dem Aromateneinsatzmaterial umgesetzt wird.

4. Verfahren nach Anspruch 1, bei dem die Transalkylierungsreaktion von Schritt (d) in zumindest teilweise flüssiger Phase bei Temperaturen zwischen 150°F (66°C) und 900°F (482°C) und Drücken zwischen 150 psig (1034 kPaÜ) und 2000 psig (13788 kPaÜ) durchgeführt wird.

5. Verfahren nach Anspruch 1, bei dem der Transalkylierungskatalysator (die Transalkylierungskatalysatoren) mindestens ein saures Feststoff-Oxid ausgewählt aus der Gruppe bestehend aus Zeolith Beta, Zeolith Y, ZSM-5, PSH-3, ITQ-2, ZM-12, MCM-22, MCM-36, MCM-49, MCM-56, MCM-58, MCM-68, Faujasit, Mordenit, porösen kristallinen Magnesiumsilicaten und Wolframat-modifiziertem Zirkonoxid umfassen.

6. Verfahren nach Anspruch 1, bei dem insgesamt das Gewichtsverhältnis von Aromaten zu Polyalkylaromaten, das in die Transalkylierungszone eingespeist wird, zwischen 0,2:1 und 20:1 liegt.

7. Verfahren nach Anspruch 6, bei dem die Aromaten Benzol sind.

8. Verfahren nach Ansprüchen 1 bis 6, bei dem das Olefin ausgewählt ist aus der Gruppe bestehend aus Ethylen und Propylen.

9. Verfahren nach Ansprüchen 1 bis 6, bei dem die Monoalkylaromaten ausgewählt sind aus der Gruppe bestehend aus Ethylbenzol und Cumol.

10. Verfahren nach Ansprüchen 1 bis 6, bei dem die Polyalkylaromaten ausgewählt sind aus Polyethylbenzol und Polyisopropylbenzol.

## Revendications

1. Procédé pour produire un produit aromatique monoalkylé dans un réacteur ayant une zone d'alkylation en série avec une zone de trans-alkylation, le procédé comprenant les étapes de procédé suivantes :
(a) introduction dans la zone d'alkylation, par deux ou plus de deux charges de composants individuels ou une ou plusieurs charges au moins partiellement combinées, d'un mélange réactionnel comprenant des aromatiques frais et recyclés et de l'oléfine fraîche, le rapport molaire des aromatiques à l'oléfine dans le mélange dépassant 1/1, et également dans lequel la zone d'alkylation comprend un ou plusieurs catalyseurs d'alkylation appropriés comprenant au moins un oxyde d'acide solide choisi dans le groupe constitué par MCM-22, MCM-36, MCM-49, MCM-56, MCM-58, MCM-68, et la zircone modifiée par du tungstate ;
(b) mise en contact du mélange d'aromatiques/oléfine avec le ou les catalyseurs d'alkylation dans des conditions d'alkylation suffisantes pour que pratiquement toute l'oléfine réagisse dans le mélange en aromatiques monoalkylés et aromatiques polyalkylés, pour produire un effluent provenant de la zone d'alkylation comprenant des aromatiques monoalkylés et polyalkylés et les aromatiques n'ayant pas réagi ;
(c) introduction d'une charge dans une zone de trans-alkylation, la charge comprenant l'effluent provenant de la zone d'alkylation, des polyalkylaromatiques recyclés, et éventuellement des aromatiques additionnels, la zone de trans-alkylation comprenant un catalyseur de trans-alkylation approprié ;
(d) mise en contact de la charge pour la zone de trans-alkylation avec le catalyseur de trans-alkylation dans des conditions de trans-alkylation suffisantes pour qu'au moins une partie des aromatiques et des aromatiques polyalkylés dans la charge réagisse aux aromatiques monoalkylés additionnels pour produire un effluent issu de la zone de trans-alkylation comprenant les aromatiques monoalkylés souhaités et les aromatiques et aromatiques polyalkylés n'ayant pas réagi ;
(e) introduction de l'effluent de la zone de trans-alkylation dans une zone de séparation dans laquelle le produit aromatique monoalkylé souhaité est isolé et récupéré et les aromatiques et aromatiques polyalkylés n'ayant pas réagi sont récupérés et recyclés.

2. Procédé selon la revendication 1, dans lequel la réaction d'alkylation de l'étape (b) est effectuée en phase liquide au moins partielle à des températures comprises entre 66°C (150°F) et 482°C (900°F) et sous des pressions comprises entre 1034 kPa au manomètre (150 psig) et 13788 kPa au manomètre (2000 psig).

3. Procédé selon la revendication 1, dans lequel le rapport molaire global des aromatiques à l'oléfine introduits dans la zone d'alkylation est compris entre 1/1 et 20/1 de façon que l'oléfine introduite dans la zone d'alkylation réagisse pratiquement complètement avec la charge d'aromatiques.

4. Procédé selon la revendication 1, dans lequel la réaction de trans-alkylation de l'étape (d) est effectuée en phase liquide au moins partielle à des températures comprises entre 66°C (150°F) et 482°C (900°F) et sous des pressions comprises entre 1034 kPa au manomètre (150 psig) et 13788 kPa au manomètre (2000 psig).

5. Procédé selon la revendication 1, dans lequel le ou les catalyseurs de trans-alkylation comprennent au moins un oxyde d'acide solide choisi dans le groupe constitué par : la zéolite bêta, la zéolite Y, ZSM-5, PSH-3, ITQ-2, ZSM-12, MCM-22, MCM-36, MCM-49, MCM-56, MCM-58, MCM-68, la faujasite, la mordénite, les silicates de magnésium cristallins poreux, et la zircone modifiée par du tungstate.

6. Procédé selon la revendication 1, dans lequel le rapport molaire global des aromatiques aux polyalkylaromatiques introduits dans la zone de trans-alkylation est compris entre 0,2/1 et 20/1.

7. Procédé selon les revendications 1 à 6, dans lequel les aromatiques sont du benzène.

8. Procédé selon les revendications 1 à 6, dans lequel l'oléfine est choisie dans le groupe constitué par l'éthylène et le propylène.

9. Procédé selon les revendications 1 à 6, dans lequel les monoalkylaromatiques sont choisis dans le groupe comprenant l'éthylbenzène et le cumène.

10. Procédé selon les revendications 1 à 6, dans lequel les polyalkylaromatiques sont choisis parmi le polyéthylbenzène et le polyisopropylbenzène.
